(19) Europäisches Patentamt
European Patent Office
Office européen des brevets

(11) Publication number: 0 179 443 B1

(12) EUROPEAN PATENT SPECIFICATION

(45) Date of publication of patent specification: 11.10.89

(51) Int. Cl.$^4$: C 07 C 103/50, C 07 C 102/00

(21) Application number: 85113385.0

(22) Date of filing: 22.10.85

# (54) Novel fluorine-containing amine amides and preparation thereof.

(30) Priority: 22.10.84 JP 222467/84

(43) Date of publication of application:
30.04.86 Bulletin 86/18

(45) Publication of the grant of the patent:
11.10.89 Bulletin 89/41

(84) Designated Contracting States:
DE FR GB

(56) References cited:
EP-A-0 136 668
GB-A-2 005 264

CHEMICAL ABSTRACTS, vol. 58, no. 8, 1963, column 7819 f-g, Columbus, Ohio, US; V. WEINMAYR "Hydrogen fluoride as a condensing agent. VI. Reactions of fluoro olefins with formaldehyde in hydrogen fluoride", & JOURNAL OF ORGANIC CHEMISTRY, 1963, 28, 492-494

(73) Proprietor: DAIKIN INDUSTRIES, LIMITED
No. 1-12-39, Umeda Kita-ku
Osaka-shi Osaka-fu (JP)

(72) Inventor: Ohsaka, Yohnosuke
16-5, Shirakawa 1-chome
Ibaraki-shi Osaka-fu (JP)
Inventor: Amimoto, Yoshio
15-9, Daizoji 2-chome
Takatsuki-shi Osaka-fu (JP)
Inventor: Negishi, Yoshio
21-21, Hitotsuya 2-chome
Settsu-shi Osaka-fu (JP)

(74) Representative: Eitle, Werner, Dipl.-Ing. et al
Hoffmann, Eitle & Partner Patentanwälte
Arabellastrasse 4
D-8000 München 81 (DE)

EP 0 179 443 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

FIELD OF THE INVENTION

The present invention relates to novel fluorine-containing amine amide and preparation thereof.

DETAILED DESCRIPTION OF THE INVENTION

The novel amine amide according to the present invention is represented by the formula:

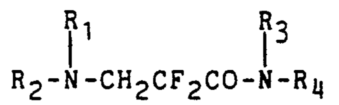

(I)

wherein $R_1$, $R_2$, $R_3$ and $R_4$ are, the same or different, a hydrogen atom or a monovalent aliphatic, alicyclic, aromatic or aralkyl group or $R_1$ and $R_2$ and/or $R_3$ and $R_4$ form a cyclic group together with the nitrogen atom to which they are bonded.

In the formula (I), the monovalent organic group may be an aliphatic, alicyclic or aromatic group. The aliphatic group may be a straight or branched group. The alicyclic or aromatic group may have at least one substituent.

According to a preferred embodiment of the invention the organic group may be a $C_1$—$C_7$ alkyl group; a phenyl group optionally having at least one substituent selected from the group consisting of a hydrocarbon group, a hydroxyl group and a hydrocarbon oxy group; a benzyl group; a cyclohexyl group; —$CH_2CF_2CONXY$; or —CO—$CF_2CH_2$—Z in which X, Y and Z are each a hydrocarbon group and their corresponding divalent groups.

The amine amide (I) may be prepared by reacting a monoamine with a 2,2,3,3-tetrafluorooxetane (hereinafter referred to as "tetrafluorooxetane") of the formula:

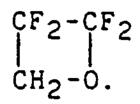

(II)

in a suitable solvent. The reaction is preferably carried out in the presence of a base or a basic salt to neutralize hydrogen fluoride liberated during the reaction.

The solvent is preferably one stable against the base. Specific examples of the solvent are diethyl ether, tetrahydrofuran, methylene chloride, 1,1,2-trichlorotrifluoroethene, bezene, toluene, diethyleneglycol dimethyl ether and the like.

Specific examples of the base and the basic salt are hydroxides of alkali metals or alkaline earth metals (e.g., sodium hydroxide, potassium hydroxide, calcium hydroxide, etc.) and salts of alkali metals with weak acids (e.g., sodium carbonate, potassium carbonate, etc.).

The reaction temperature is usually from a room temperature to a reflux temperature of the solvent, preferably up to a temperature generated by a reaction heat.

The monoamine may be any one of known monoamines. Since the reaction proceeds between the amino group of the monoamine and tetrafluorooxetane, it is not affected by the kinds of the groups $R_1$, $R_2$, $R_3$ and/or $R_4$. Thus, these groups may be any aliphatic or aromatic, or substituted or unsubstituted ones. Any amino acid can be used as the monoamine according to the present invention. Further, these groups may be ones having a hetero atom such as a polyether group. $R_1$ and $R_2$ and/or $R_3$ and $R_4$ may form a cyclic group together with the nitrogen atom of the amino group. Further, the reaction according to the present invention is not influenced by the chain length of these groups.

Tetrafluorooxetane is a known compound and may be prepared by reacting tetrafluoroethylene and paraform in anhydrous hydrogen fluoride.

The fluoride-containing amine amides according to the present invention are useful as a thickening agent, a disoxidation catalyst and a monomer. In addition, some of them may be used as color display components.

The present invention will be hereinafter explained further in detail by following examples.

### Examples 1 to 6

To a mixture of a following amine (0.4 mole) and glyme (40 ml), a mixture of tetrafluorooxetane (9 ml) and glyme (20 ml) was dropwise added with stirring. After the ceasing of exothermic reaction, the reaction mixture was cooled to around a room temperature and filtered by a suction filter. After removing glyme from the filtrate, the residue was washed with water and dried to obtain a following amine amide.

(Example 1)

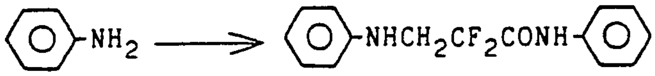

Yield 100%.
IR (KBr): 1,680 $cm^{-1}$ (C=O), 3,420 and 3,340 $cm^{-1}$ (N—H).
$^{19}F$—NMR: 31.7 ppm (td, 14.3 Hz).
$^{1}H$—NMR: δ= 3.89 (2H, t, 13 Hz), 3.9 (1H, br, s), 6.65—6.85 and 7.1—7.6 (10H, m) and 8.1 (1H, br, s).

(Example 2)

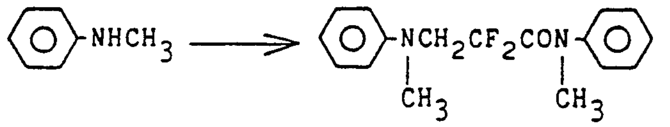

Yield 36%.
IR (KBr): 1,660 $cm^{-1}$ (C=O).

(Example 3)

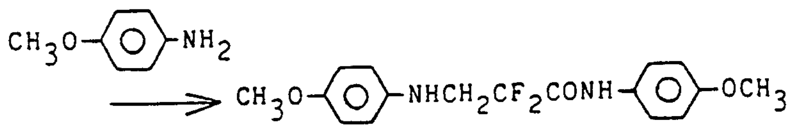

Yield 84%.
IR (KBr): 1,670 $cm^{-1}$ (C=O), 3,380 and 3,330 $cm^{-1}$ (N—H).

(Example 4)

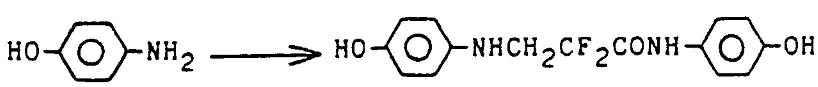

Yield 90%.
IR (KBr): 1,680 $cm^{-1}$ (C=O), 3,370 and 3,300 $cm^{-1}$ (N—H, O—H).

(Example 5)

$$HOCH_2CH_2NH_2 \longrightarrow OHCH_2CH_2NHCH_2CF_2CONHCH_2CH_2OH$$

Yield 42%.
IR (neat): 1,680 $cm^{-1}$ (C=O) and 3,300 $cm^{-1}$ (N—H, O—H).

(Example 6)

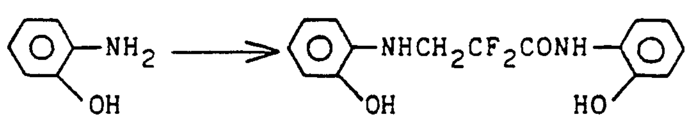

Yield 100%.
IR (neat): 1,680 $cm^{-1}$ (C=O), 3,300 $cm^{-1}$ (N—H, O—H).

Examples 7, 8 and 9

To a mixture of a following amine (0.1 mole), an aqueous potassium hydroxide solution (5.7 g/50 ml) and diethyl ether (50 ml), a mixture of tetrafluorooxetane (4.5 ml, 0.05 mole) and diethyl ether (25 ml) was dropwise added with stirring. After the completion of the addition, the reaction mixture was stirred for another 2 hours. Then, the ether layer was separated and dried. By evaporating the ether or by filtering the precipitated product following neutralization, the product was reocvered, washed with water and dried to obtain a following amine amide.

(Example 7)

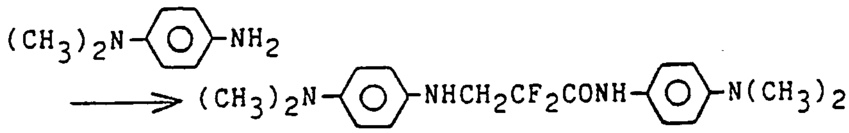

Yield 66%.
IR (KBr): 1,670 $cm^{-1}$ (C=O), 3,340, 3,320 and 3,260 $cm^{-1}$ (N—H).

(Example 8)

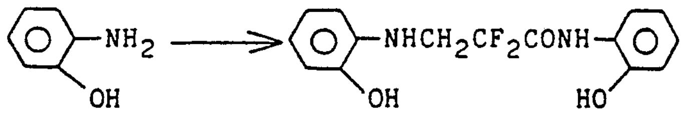

Yield 87%.

(Example 9)

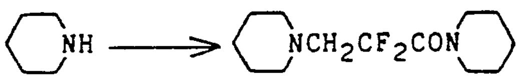

Yield 100%.
IR (neat): 1,680 $cm^{-1}$ and 1,660 $cm^{-1}$ (C=O).

Example 10

To a solution of a following amino acid (0.1 mole) in an aqueous solution of potassium hydroxide (8.0 g/50 ml), a mixture of tetrafluorooxetane (4.5 ml) and diethyl ether (25 ml) was dropwise added. After the ceasing of the exothermic reaction, the reaction mixture was cooled to around a room temperature and filtered. To the filtrate, calcium chloride (5.56 g, 0.05 mole) was added, kept overnight and filtered. To the filtrate, concentrated hydrochloric acid (12 ml) was added to precipitate the product, which was washed with water and dried to obtain a following amine amides.

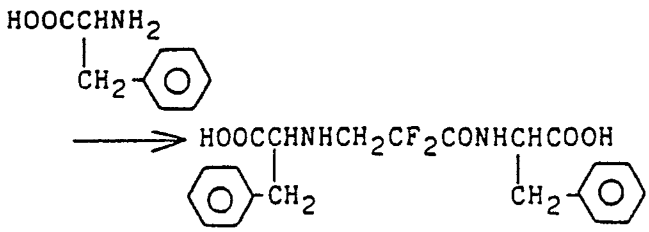

Yield 54%.
IR (KBr): 1,680 $cm^{-1}$ (C=O) and 3,330 $cm^{-1}$ (N—H).

and

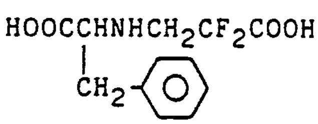

Yield 9%.
IR (KBr): 1,750 and 1,640 $cm^{-1}$ (C=O) and 3,400 $cm^{-1}$ (N—H).

## Claims

1. An amine amide of the formula:

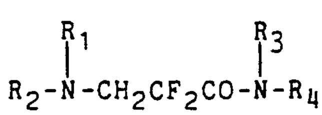
(I)

wherein $R_1$, $R_2$, $R_3$ and $R_4$ are, the same or different, a hydrogen atom or a monovalent aliphatic, alicyclic, aromatic or aralkyl group or $R_1$ and $R_2$ and/or $R_3$ and $R_4$ form a cyclic group together with the nitrogen atom to which they are bonded.

2. A process for preparing an amine amide of the formula:

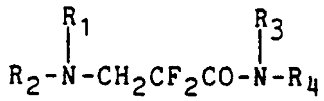

(I)

wherein $R_1$, $R_2$, $R_3$ and $R_4$ are, the same or different, a hydrogen atom or a monovalent aliphatic, alicyclic, aromatic or aralkyl group or $R_1$ and $R_2$ and/or $R_3$ and $R_4$ form a cyclic group together with the nitrogen atom to which they are bonded, comprising reacting a monoamine with a 2,2,3,3-tetrafluorooxetene of the formula:

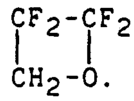

(II)

3. A process according to claim 2, wherein the reaction is carried out in a solvent.

4. A process according to claim 3, wherein the solvent is one selected from the group consisting of diethyl ether, tetrahydrofuran, methylene chloride, 1,1,2-trichlortrifluoroethene, benzene, toluene and diethyleneglycol dimethyl ether.

5. A process according to claim 2, wherein the reaction is carried out in the presence of a base or a basic salt.

6. A process according to claim 5, wherein the base is an alkali metal hydroxide.

7. A process according to claim 5, wherein the base is an alkaline earth metal hydroxide.

8. A process according to claim 5, wherein the basic salt is a salt of an alkali metal with a weak acid.

**Patentansprüche**

1. Aminamid der Formel

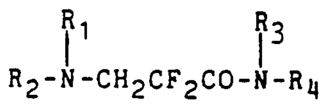

(I)

wobei $R_1$, $R_2$, $R_3$ und $R_4$ gleich oder verschieden sind und ein Wasserstoffatom oder eine monovalente aliphatische, alizyklische, aroamtische oder Aralkylgruppe sind, oder $R_1$ und $R_2$ und/oder $R_3$ und $R_4$ eine zyklische Gruppe zusammen mit dem Stickstoffatom, an das sie gebunden sind, bilden.

2. Verfahren zur Herstellung eines Aminamids der Formel

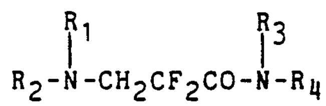

(I)

wobei $R_1$, $R_2$, $R_3$ und $R_4$ gleich oder verschieden sind und ein Wasserstoffatom oder eine monovalente aliphatische, alizyklische, aromatische oder Aralkylgruppe darstellen, oder $R_1$ und $R_2$ und/oder $R_3$ und $R_4$ eine zyklische Gruppe zusammen mit dem Stickstoffatom, an das sie gebunden sind, bilden, das die Reaktion eines Monoamins mit 2,2,3,3-Tetrafluoroxetan der Formel

(II)

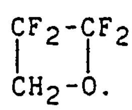

umfasst.

3. Verfahren nach Anspruch 2, wobei die Reaktion in einem Lösungsmittel ausgeführt wird.

4. Verfahren nach Anspruch 3, wobei das Lösungsmittel aus der Reihe Diethylether, Tetrahydrofuran, Methylenchlorid, 1,1,2-Trichlortrifluorethen, Benzol, Toluol und Diethylenglykoldimethylether ausgewählt wird.

5. Verfahren nach Anspruch 2, wobei die Reaktion in Gegenwart einer Base oder eines basischen Salzes durchgeführt wird.

6. Verfahren nach Anspruch 5, wobei die Base ein Alkalimetallhydroxid ist.

7. Verfahren nach Anspruch 5, wobei die Base ein Erdalkalimetallhydroxid ist.

8. Verfahren nach Anspruch 5, wobei das basische Salz ein Salz eines Alkalimetalls mit einer schwachen Säure ist.

**Revendications**

1. Un amine-amide de formule:

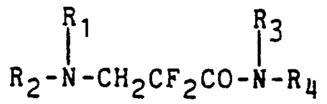

(I)

dans laquelle $R_1$, $R_2$, $R_3$ et $R_4$ sont identiques ou différents, et représentent un atome d'hydrogène ou un groupe monovalent aliphatique, alicyclique, aromatique ou un groupe aralkyle ou bien $R_1$ et $R_2$ et/ou $R_3$ et $R_4$ forment un groupe cyclique ensemble avec l'atome d'azote auquel ils sont attachés.

2. Une procédé de préparation d'un amine-amide de formule:

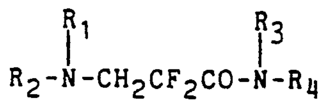

(I)

dans laquelle $R_1$, $R_2$, $R_3$ et $R_4$ sont identiques ou différents et représentent un atome d'hydrogène, ou un groupe monovalent aliphatique, alicyclique, aromatique ou un groupe aralkyle ou bien $R_1$ et $R_2$ et/ou $R_3$ et $R_4$ forment un groupe cyclique ensemble avec l'atome d'azote auquel ils sont attachés, comprenant la réaction d'une monoamine avec un 2,2,3,3-tétrafluorooxétanne de formule:

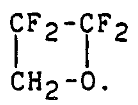

(II)

3. Une procédé selon la revendication 2, selon lequel la réaction est conduite dans un solvant.

4. Un procédé selon la revendication 3, selon lequel le solvant est choisi dans le groupe comprenant l'éther diéthlique, le tétrahydrofuranne, le chlorure de méthylène, le 1,1,2-trichlorotrifluoroéthène, le benzène, le toluène et l'éther diméthlique du diéthlèneglycol.

5. Un procédé selon la revendication 2, selon lequel la réaction est conduite en présence d'une base ou d'un sel basique.

6. Un procédé selon la revendication 5, selon lequel la base est un hydroxyde de métal alcalin.

7. Un procédé selon la revendication 5, selon lequel la base est un hydroyde de métal alcalino-terreux.

8. Un procédé selon la revendication 5, selon lequel la sel basique est un sel d'un métal alcalin avec un acide faible.